# EUROPEAN PATENT SPECIFICATION

(11) **EP 2 107 936 B1**
(45) Date of publication and mention of the grant of the patent: **01.08.2012**
(21) Application number: 07862094.5
(22) Date of filing: 16.11.2007
(51) Int. Cl.: A61L 2/20, B01J 19/08, B01J 19/12, A23L 3/34, A23B 7/152

(54) **SHIPPING CONTAINER OZONATION SYSTEM**
SYSTEM ZUR OZONISIERUNG VON TRANSPORTBEHÄLTERN
SYSTÈME DE TRAITEMENT À L'OZONE DE CONTENEUR D'EXPÉDITION

(30) Priority: 29.12.2006 US 648711
(43) Date of publication of application: 14.10.2009
(73) Proprietor: Purfresh, Inc., Livermore, CA 94551 (US)
(72) Inventor: DICK, Paul, Fremont, CA 94538 (US); COPE, David, Fremont, CA 94538 (US); WANG, Howard, Fremont, CA 94538 (US); HOOBLER, Ray, Fremont, CA 94538 (US); WEBER, Michael, Fremont, CA 94538 (US); VOLONDIN, Andrew, Fremont, CA 94538 (US)
(74) Representative: Käck, Jürgen
(86) International application number: PCT/US2007/024102
(87) International publication number: WO 2008/082452

(56) References cited:
- WO-A1-02/074349
- JP-A- 4 311 345
- US-A- 3 521 459
- US-A- 3 719 564
- US-A- 5 594 740
- US-A- 6 019 949
- US-A- 6 086 833
- US-A1- 2005 123 436
- US-B1- 6 365 026
- US-B1- 6 502 409
- US-B1- 6 615 908

## Description

### BACKGROUND OF THE INVENTION

### Field of the Invention

This invention relates to transport equipment for perishable foods, and more specifically to refrigerated containers for shipping fresh or frozen food products.

### Description of the Prior Art

US3521459 discloses a fresh produce storage cargo container, which is cooled, sterilized by ozone and a portion exchanged with the outside atmosphere. The mixture passes through a moisture zone below the produce at the bottom of the storage chamber which receives condensate form the cooler, the moist air rising through the chamber and so round again.

WO02074349 discloses a fumigant system for transportation containers comprising an ozone generator and sensor, roller, a fan.

JP4311345 discloses a freshness keeping apparatus with a controller, a passage for circulating freshness-retaining gas, containing a section where the gas is irradiated with the 184.9nm UV at first and with 253.7nm UV by a mercury lamp and the section where ethylene oxide is eliminated, a detector) for ozone installed near the outlet of the passage and a controller having a medium for determining the output of the mercury lamp and an output medium.

US2005123436 discloses the use of ozone treatment with specific combination of ozone concentration, temperature and humidity for abating a pathogen, allergen or odour.

Perishable agricultural products such as harvested fruits, vegetables, and flowers, as well as frozen foods, are typically transported in refrigerated shipping containers. These containers are designed for transport by truck, rail, air, or ship, enabling consumers to enjoy a wide variety of products year-round from many parts of the world.

Refrigeration itself is an effective means of preserving the freshness of agricultural products for extended periods of time and of inhibiting spoilage and the growth of microorganisms. However, refrigeration only retards the growth of these microorganisms and does not destroy them, and as much as 20% of all product shipped worldwide is lost to spoilage and rot. Further, certain fresh products emit ethylene, which promotes undesired ripening of the products during transport.

Additional preservation of freshness is accomplished by storing and transporting the product in a controlled atmosphere (CA) environment, which slows respiration and ripening of the fruit, leading to increased shelf life. In a CA environment, the atmospheric air normally surrounding the product is replaced by a gas that contains a reduced oxygen level (generally with a target of about 1-5% compared to about 21% in air), with the balance mostly nitrogen and CO2 produced by respiration.

Further protection may be obtained by exposing the products to ozone. Ozone destroys microorganisms rather than simply retarding their growth. Ozone also decomposes the ethylene that fresh products emit, thereby retarding the ripening of the products by reducing their exposure to ethylene in the atmosphere inside the container. Ozone that is not consumed in reactions readily decomposes, leaving no residue. However, because the ozone gas used in refrigerated environments decomposes over time, the efficacy of its use is limited by both the decomposition rate and the difficulty of achieving and maintaining the desired ozone level in the atmospheric air or CA gas surrounding the products.

An ozone generator according to the current art typically has a control panel that is used to turn ozone generation on and off and to adjust the amount of ozone generated by setting the level of electrical power supplied to the generator. However, a typical ozone generator does not include a means for measuring the amount of ozone delivered to the product, and even when run by an experienced operator the ozone may be delivered in too high a concentration. As a result, product may be "burned", bleached or otherwise damaged by too high an ozone dose. On the other hand, ozone doses that are too low or not applied at the right time do not result in adequate product preservation.

Many parameters of the container environment such as temperature, humidity, ethylene concentration or CO2 concentration are critical during the ripening process and also affect the optimal doses of ozone. These parameters change dynamically. While in the prior art it is possible to monitor and control temperature and humidity of the gaseous atmosphere in the container, there appears to be no prior art system for monitoring and altering the composition of gases in the environment to control the state of the product. Also, a typical prior art ozone generator does not have means to adjust the ozone delivery to optimize preservation of the product based on real-time determination of these parameters or according to a pre-defined program.

In the current art, an operator activates and monitors ozone production directly at the generator. This is cumbersome with a container that may be stacked in an inaccessible location in a container yard, and may be impossible if the container is traveling on a truck or boat. Also, it is not possible to obtain a status and record of the ozone application and other product-related parameters without direct access to the ozone generator. No real time data is available, and log data can only be downloaded after the trip is complete. Thus, the state of the ozonation system or the product cannot be detected and acted upon prior to the end of the journey.

In the current art, ozone is injected at all times, whether the circulation system of the container is running or not. In some cases the circulation system may shut down while the refrigeration unit is defrosted or temporarily looses power. In such a situation, ozone of high concentration (>1ppm) continues to be admitted into the container and can come in contact with the product causing damage and loss.

A refrigerated container runs on electrical power and contains a control panel which allows for refrigeration-related parameters of operation such as temperature, defrost cycles, and so on to be preset. However, in order to maintain cooling and preserve the product, the container must be continuously connected to electrical power. In the current art, there is again no way to monitor the container once it has been loaded and is on its voyage, or to determine whether there is a problem with the cooling system such as a loss of power or component failure. It is not possible to remotely monitor and control the environment inside the container (including temperature; humidity; power status; refrigeration system function; oxygen, carbon dioxide, ethylene or ozone levels; door status; air exchange status) or to determine the location of the container anywhere in the world.

### SUMMARY OF THE INVENTION

The invention is defined by claim 1.

Other embodiments of the invention may also include sensors for sensing parameters of the recirculating gas, the product and the container itself other than ozone concentration, such as gas temperature and humidity, 02, CO2 and ethylene concentrations; product temperature, product color, container door status, container power, container location, airborne particles,and/or circulation system operational status.

The control system may control the timing, amount and/or location of ozone injection based on a "recipe" which includes rules based on sensor readings or a predetermined profile. Some embodiments of the invention also have safeguards and interlocks to protect product and workers; for example, ozone production may be stopped when the circulation system stops.

Embodiments of the invention may also include communication means to communicate with external devices in order to load recipes for ozonation control, turn the ozone on or off, set levels, and/or download status and sensor readings. The communication means may be wired or wireless, and may also include an antenna and a device to establish a local area (short-range) wireless network connection, communicate via a cellular network, or communicate via a satellite link.

### BRIEF DESCRIPTION OF THE DRAWINGS

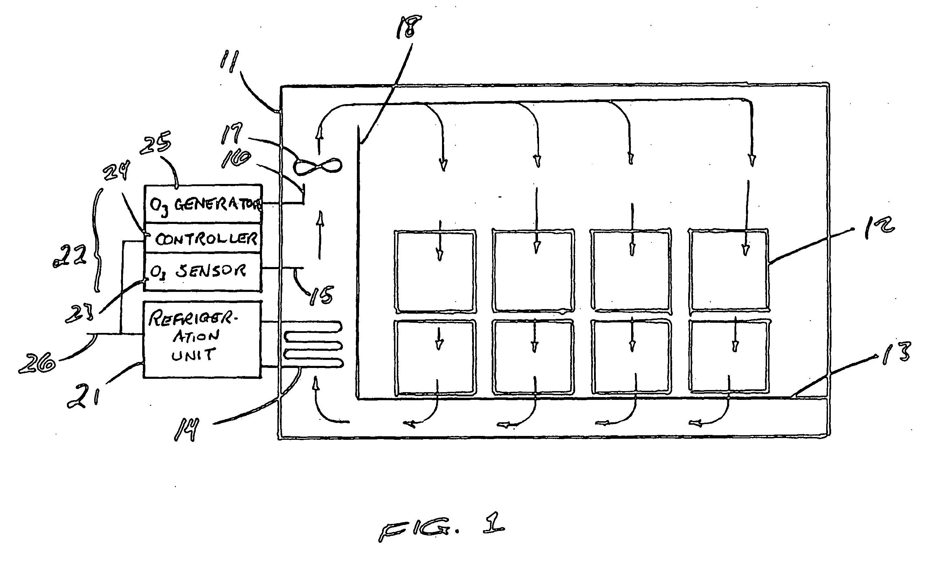

FIG. 1 is a diagram showing a vertical cross section of a shipping container in accordance with one embodiment of the present invention.

### DETAILED DESCRIPTION OF THE INVENTION

The present invention describes shipping containers that are useful in the transportation of any food products that are susceptible to spoilage over extended periods of time and for which the rate of spoilage can be reduced by refrigeration, ozone treatment, or both. While fruits and vegetables are of particular interest, the present invention may also be used with other products, including, but not limited to, fresh produce and other agricultural products, cooked foods, frozen foods (both cooked and uncooked) and other products and materials that will be readily apparent to one of skill in the food shipping industry.

The containers themselves can vary widely depending on the mode of transportation. Examples of containers include rail cars, truck trailers, and containers loaded on ocean-going ships. The present invention is particularly suited to containers that are fully self-contained, with the ozone sensor, controller, and injector either residing in the interior of the container or attached in whole or in part to the outer surface of the container. However, the present invention may be used with other containers as well, such as the hold of a cargo ship, or a refrigerated trailer with permanently mounted axes and wheels.

In addition to a refrigeration system, the container incorporates a circulation system for the gaseous atmosphere within the container that includes a fan. The gaseous atmosphere may be of the RA (regular atmosphere) type or the CA (controlled atmosphere) type. In RA, the recirculating gas has an oxygen level similar to regular atmosphere (about 21 %). The CA environment is typically depleted in oxygen, with typical levels ranging from 1% to 5% but sometimes as high as 15%, and enriched in nitrogen and CO2. The circulation system may also include a vent opening that can be adjusted from closed (no mixing with air in the external environment) to open (high rate of replacement of recirculating air with fresh air from the external environment). Air exchange allows the reduction by dilution of undesired gases such as ethylene.

The container also includes an ozone generator, which consists of at least one ozone module including an ozone-generating cell and an appropriate power supply. Additional ozone modules may be provided for increased ozone output or redundancy in the case of failure of a generator module. Ozone injectors that may be used in the practice of the invention include ozone generators that convert gaseous oxygen to ozone by corona discharge. Such ozone generators utilizing corona discharge are known and commercially available. Examples include those described in Ditzler, L.C., United States Patent No. 4,877,588, issued October 31,1989, Ditzler, L.C., et al., United States Patent No. 5,945,073, issued August 31, 1999, and Ditzler, L.C., et al., United States Patent No. 6,372,096, issued April 16, 2002 . One embodiment of the invention utilizes two generators in parallel, both of which are of the corona discharge type.

The oxygen may be supplied as air or oxygen-enriched air, with the generator forming an ozone-oxygen or ozone-air mixture. Having the air or oxygen-enriched air be dry air may enhance the level of ozone production in the generator.

The ozone generator may also include an air treatment system for the air supply to the ozone module(s),which is used to eliminate particles and perform other useful functions, for example, reduction of water vapor and increase of oxygen concentration in the intake air. Particles may clog components and reduce ozone output, while water has detrimental effects on ozone generation such as reduced output and corrosion. An increased oxygen concentration leads to increased ozone concentration and higher output, which is desirable. In one embodiment, the air treatment system comprises a particle filter, and may include an absorbent trap, a cold trap operated with a thermo-electric cooler, a condensing trap, or a PSA (pressure-swing absorption) device to change the oxygen concentration and decrease water vapor.

An ozone injector is also provided for injecting ozone into the gas recirculating in the container. It is desirable to inject the ozone in a way that creates a reaction area of relatively high concentration, as well as uniformly mixes the ozone with the recirculating gas, so that ethylene-destroying reactions can occur. Accordingly, the ozone may be injected at two or more points. In one embodiment, the injection is done in close proximity above or below each of the fans in the circulation system. The injector may have just one nozzle (i.e., an opening for ozonated gas to exit), or multiple nozzles arranged across the fan cross section. For example, the injector could have a bar with multiple nozzles, or a circular piece of tubing with multiple nozzles.

In some embodiments, the container may also have means to dilute the injected ozone, which typically has a high concentration (over 1 ppm) in the gas mixture exiting the ozone generator. This dilution may be accomplished by mixing the ozonated gas with a volume of the gas inside the container, or with a volume of air or CA gas introduced into the container. In one embodiment, the means for dilution is the main circulation of the container refrigeration system, i:e., the ozone is injected into the recirculating gas stream. In other embodiments, the ozone distribution system may include other dilution means such as additional fans, venturis, nozzles, or a makeup gas line connected to the output of the ozone generator. The input and output of the ozone-generating cell may be in-line with the recirculating gas stream, with or without a secondary means to force gas through the cell. The ozone-generating cell intake line may also be connected directly to the air mix intake line of the container.

The amount of ozone present in the resulting ozone-enriched air should vary with the needs of the system, based upon factors including the nature of the product stored in the container, the volumetric capacity of the container, and the rate of air circulation within the container. In many cases, appropriate rates of ozone generation are those that will achieve an ozone concentration level of between approximately 80 ppb and 1.2 ppm, by volume, in the injected mixture, with the injected mixture having a volumetric flow rate of between approximately I and 20 cubic feet per hour (about 0.5 to about 10 liters per minute). Corona discharge ozone generators can typically generate ozone in a concentration range of between approximately 0.1 % and about 10% by weight with a production rate between approximately 0.01 and 10.0 grams per hour.

To maintain the desired ozone concentration, the ozone in the circulating gas stream may be measured by an ozone sensor. A variety of ozone sensors are available and may be used in various embodiments. One type of commonly used ozone sensor utilizes gas-phase electrochemical cells, while others are solid-state sensors utilizing metal composites, metal oxides, or organic polymers, and still others are based on chemiluminescence or absorption of ultraviolet light. Electrochemical cell-type sensors may yield the best results because of their sensitivity and small size.

In one embodiment, the ozone sensor is located downstream of the point where ozone is injected, and upstream of the area where the recirculating gas first comes into contact with the product. This arrangement allows for testing of the ozonated gas stream before it contacts the product to insure that the ozone concentration does not exceed damage-causing limits. At the same time, a higher amount of ozone can be added to enhance the ethylene destruction in the injection area.

The container incorporates an automated controller that uses the measured ozone concentration to govern the rate of ozone added to the circulating gas by the injector so that a desired ozone level is maintained in accordance with a preset target value or concentration profile. The invention includes means for the controller to determine the status of the recirculation fans and reduce or shut off ozone production or injection, in particular when a recirculating fan is off. The ozone generator or controller is connected to the same power supply as the fan power, automatically shutting off power and thus ozone production when the fan is not powered. In other embodiments, a sensor or switch that senses fan operation or air movement is in communication with the ozonation system, and ozonation is reduced or stopped when there is no recirculation.

The controller may also be in communication with the air vent system of the main refrigeration system and be able to adjust the air mix to accomplish a dilution of undesired gases such as ethylene and CO2. This may be accomplished by, for example, a remote actuator for the air vent dial or by valves.

As above, the controller may accept signals from the ozone sensor, compare the signals to a programmed target, and emit a corresponding signal to the injector. In some embodiments, the target ozone concentration may be within the range of approximately 0.01 ppm to about 5.0 ppm, by volume, and a range of approximately 0.55 ppm to 2.0 ppm, also by volume, may be particularly effective. When the perishable products in the container include those that emit ethylene, the demand for ozone will be initially high but then decrease as the ethylene is decomposed. The target ozone concentration in these cases can therefore decrease with time to correspond to the amount of ethylene present. In other cases, the ozone demand may initially be fulfilled with a low concentration but ozone concentration is increased when increased emission of ethylene or CO2 is detected. In still other cases, the target ozone concentration may be an oscillating or pulsed value, for example alternating between a high level of approximately 1.0 to 5.0 ppm and a low level that is at least approximately 0.5 ppm lower than the high level, again all by volume.

All of these target concentrations may be readily achieved with programmable controllers. Examples include PLCs (programmable logic controllers) such as those manufactured by Allen-Bradley. Alternatively, computers with readily available instrumentation control software, such as LabView from National Instruments Corporation, may be used. It is desirable to have a controller of small size; for example, the controller may be based on an embedded microprocessor with integrated input/output functions such as the PIC processor manufactured by Microchip Technologies. Certain controllers may also record the ozone concentration as a function of time and retain the record in a readily accessible format. Remote monitoring of the ozone concentration and other parameters may be achieved by combining the programmable controller with known monitoring and transmission systems, such as global positioning systems, cellular telephone systems, satellite telephone systems, and other systems known in the art.

As above, the circulating gas is typically an oxygen-containing gas, and most conveniently air, and ozone is generated from the oxygen in the circulating gas by drawing the gas into a corona discharge ozone generator or other ozone generator. The circulation rate of the circulating gas may vary according to the needs of the container and of the product transported in the container. In some applications, a circulation rate of approximately 100 to 10,000 cubic feet per minute (about 50 to about 5000 liters per second) may provide the best results. (All conversions to the metric system in this specification are rounded off to the nearest round number.) In some cases, air, possibly containing decomposition products of the microorganisms or ethylene, may be released from the container and replaced with other air. When this occurs, the volumetric flow rate of replacement air may range from approximately I to about 50 cubic feet per minute (about 0.5 to about 25 liters per second).

In other embodiments of the invention, additional sensors can also be included that measure the state of the container and the state of ripening of the product by measuring various factors such as humidity, temperature, color of the product, and levels of carbon dioxide, molecular oxygen, ethylene, ammonia, and other contaminants or emitted gases in the recirculating gas stream. This information may be used by the controller to adjust ozone concentration and other parameters of the container environment including temperature, humidity and air mix.

The container may also include an antenna and a wired or wireless transmitting means in communication with the ozone controller. The wireless transmitting means may include one channel of wireless communication, for example, satellite, cellular network, or local area wireless network communications, and a means for determining position which, in one embodiment, is a GPS receiver.

When an antenna and transmitting means is provided, the container may be in communication with a stationary base. The stationary base may have a storage means for storing data transmitted by the container and may also incorporate a means for sending out control signals. The signal means may perform other functions as well, for example downloading recipes based on information about the product and its shipment route, notifying personnel of events including malfunctions or failures of the equipment, generating quality control information including trendcharts, and generating reports and billing information.

The dimensions of the container itself may vary widely. A refrigerated trailer on a truck, for example, may have a capacity of from about 1,000 to about 10,000 cubic feet (about 30 to about 300 cubic meters), while a shipping container for an ocean-going vessel, may have a capacity of from about 500 to about 5,000 cubic feet (about 150 to about 1,500 cubic meters). In some embodiments, the container has internal baffles, walls, or other flow-control guides to direct the circulating gas stream through all portions of the container so that all the products are exposed to gas flowing at a substantially uniform rate and so that the injected ozone is substantially uniformly dispersed throughout the contained interior. In other embodiments, the ozone is injected at multiple locations.

The appropriate refrigeration temperature for any particular product will be readily apparent to those in the industry, and particularly those skilled in the art of shipping perishable foods and other products. The temperature generally depends on the product, and temperatures for non-frozen foods typically range from approximately 0°C to about 15°C. Conventional refrigeration units that are typically used for refrigerated containers may be used.

Useful embodiments are susceptible to a wide range of variation in the configuration and size of the shipping container, the arrangement of the refrigeration and ozone-related components, and the operating conditions. An understanding of the invention as a whole may be gained from a depiction of one specific embodiment, as shown in FIG. 1 and explained below.

The shipping container **11** of FIG. **1** is an insulated box, inside which perishable products are contained in open crates **12** stacked inside the box and resting on a slotted floor **13**. Also included in shipping container **11** are a refrigeration coil **14**, an ozone sensing probe **15**, an ozone inlet **16**, and a fan **17**. The refrigeration coil **14**, ozone probe **15**, and fan **17** are separated from the crates **12** by an air flow baffle **18**. Mounted to the exterior wall of the box are a refrigeration unit **21** supplying refrigerant to the coil **14,** and a unit **22** which includes the external portion **23** of the ozone probe **15**, a controller **24**, and an ozone generator **25**. All of these units are as described above. The direction of air flow is indicated by the arrows; the circulating air or oxygen-bearing gas passes through the crates **12** and the slotted floor **13**. Power is supplied to both the refrigeration unit **21** and the ozone unit **22** through a common power supply line **26**.

The foregoing is offered primarily for purposes of illustration.

## Claims

1. A shipping container for protecting perishable items from spoilage during shipping using ozone, the shipping container containing an atmosphere and comprising:
a storage area (11) for the perishable items;
a circulation system (17) comprising a fan for circulating the atmosphere through the storage area (11) so that the atmosphere contacts the perishable items;
an ozone generator (25) providing ozone to an ozone injector (16) for adding ozone to the circulating atmosphere;
an ozone sensor (15, 23) for measuring the ozone level in the circulating atmosphere;
a controller (24) coupled to the ozone sensor (15, 23) and to the ozone injector (16), for comparing the measured ozone level with a target ozone level, and adjusting the amount of ozone added by the ozone injector (16) to achieve the target ozone level;
**characterized in**
**that** the ozone generator (25) is connected to the same power supply as the fan (17), automatically shutting off power and thus ozone production when the fan is not powered.

2. The shipping container of claim 1, further comprising a communications system for transmitting information to a remote location and receiving instructions from the remote location.

3. The shipping container of claim1, further comprising a refrigeration system (26).

4. The shipping container of claim 2, wherein the remote location is a control panel of the shipping container.

5. The shipping container of claim 2, wherein the controller is adapted to receive instructions from the remote location to start or stop the ozone injector (16), change a setpoint, or to transmit a ozonation status to the remote location.

## Patentansprüche

1. Transportbehälter zum Schützen von verderblichen Gütern vor dem Verderben während des Transports unter Verwendung von Ozon, wobei der Transportbehälter eine Atmosphäre enthält und aufweist:
einen Lagerbereich (11) für die verderblichen Güter;
ein Zirkulationssystem (17) mit einem Ventilator zum Zirkulieren lassen der Atmosphäre durch den Lagerbereich (11), so dass die Atmosphäre mit den verderblichen Gütern in Berührung kommt;
einen Ozongenerator (25), der Ozon zu einem Ozoninjektor (16) liefert, um Ozon zur zirkulierenden Atmosphäre hinzuzufügen;
einen Ozonsensor (15, 23) zum Messen des Ozonpegels in der zirkulierenden Atmosphäre;
eine mit dem Ozonsensor (15, 23) und mit dem Ozoninjektor (16) gekoppelte Steuereinheit (24) zum Vergleichen des gemessenen Ozonpegels mit einem Zielozonpegel und zum Einstellen der Menge an Ozon, das durch den Ozoninjektor (16) hinzugefügt wird, um den Zielozonpegel zu erreichen;
**dadurch gekennzeichnet, dass**
der Ozongenerator (25) mit derselben Leistungsversorgung wie der Ventilator (17) verbunden ist, wobei die Leistung und folglich die Ozonproduktion automatisch abgeschaltet wird, wenn der Ventilator nicht betrieben wird.

2. Transportbehälter nach Anspruch 1, der ferner ein Kommunikationssystem zum Übertragen von Informationen zu einem entfernten Ort und zum Empfangen von Befehlen von dem entfernten Ort aufweist.

3. Transportbehälter nach Anspruch 1, der ferner ein Kühlsystem (26) aufweist.

4. Transportbehälter nach Anspruch 2, wobei der entfernte Ort ein Bedienfeld des Transportbehälters ist.

5. Transportbehälter nach Anspruch 2, wobei die Steuereinheit dazu ausgelegt ist, Befehle vom entfernten Ort zu empfangen, um den Ozoninjektor (16) zu starten oder zu stoppen, einen Sollpunkt zu ändern oder einen Ozonierungszustand zum entfernten Ort zu übertragen.

## Revendications

1. Conteneur d'expédition destiné à protéger des éléments périssables d'une détérioration au cours de l'expédition en utilisant de l'ozone, le conteneur d'expédition contenant une atmosphère et comprenant :
une zone de stockage (11) destinée aux éléments périssables ;
un système de mise en circulation (17) comprenant un ventilateur destiné à faire circuler l'atmosphère à travers la zone de stockage (11) de sorte que l'atmosphère soit en contact avec les éléments périssables ;
un générateur d'ozone (25) fournissant de l'ozone à un injecteur d'ozone (16) destiné à ajouter de l'ozone à l'atmosphère mise en circulation ;
un capteur d'ozone (15, 23) destiné à mesurer le niveau d'ozone dans l'atmosphère mise en circulation ;
un dispositif de commande (24) couplé au capteur d'ozone (15, 23) et à l'injecteur d'ozone (16), destiné à comparer le niveau d'ozone mesuré à un niveau d'ozone cible, et à ajuster la quantité d'ozone ajoutée par l'injecteur d'ozone (16) afin d'atteindre le niveau d'ozone cible ;
**caractérisé en ce**
**que** le générateur d'ozone (25) est connecté à la même alimentation électrique que le ventilateur (17), coupant automatiquement l'alimentation et par conséquent la production d'ozone lorsque le ventilateur n'est pas alimenté.

2. Conteneur d'expédition selon la revendication 1, comprenant en outre un système de communication destiné à transmettre une information à un emplacement à distance et à recevoir des instructions provenant de l'emplacement à distance.

3. Conteneur d'expédition selon la revendication 1, comprenant en outre un système de réfrigération (26).

4. Conteneur d'expédition selon la revendication 2, dans lequel l'emplacement à distance est un panneau de commande du conteneur d'expédition.

5. Conteneur d'expédition selon la revendication 2, dans lequel le dispositif de commande est conçu pour recevoir des instructions provenant de l'emplacement à distance afin de lancer ou de stopper l'injecteur d'ozone (16), de changer un point de consigne, ou de transmettre un état d'ozonisation à l'emplacement à distance.
